# EUROPÄISCHE PATENTSCHRIFT

(11) **EP 1 234 020 B1**
(45) Veröffentlichungstag und Bekanntmachung des Hinweises auf die Patenterteilung: **19.05.2010**
(21) Anmeldenummer: 00987214.4
(22) Anmeldetag: 04.11.2000
(51) Int. Cl.: C12N 1/20, A01N 63/00, A01C 1/06

(54) **BEHANDLUNG VON SAATGUT UND PFLANZEN MIT NÜTZLICHEN BAKTERIEN**
TREATMENT OF SEEDS AND PLANTS WITH USEFUL BACTERIA
TRAITEMENT DE SEMENCES ET DE PLANTES AVEC DES BACTERIES UTILES

(30) Priorität: 29.11.1999 DE 19957378
(43) Veröffentlichungstag der Anmeldung: 28.08.2002
(73) Patentinhaber: Sourcon-Padena GmbH & Co. KG, 72072 Tübingen (DE)
(72) Erfinder: Vogt, Dr., Wolfgang, 72076 Tübingen (DE)
(74) Vertreter: Otten, Hajo
(86) Internationale Anmeldenummer: PCT/EP2000/010903
(87) Internationale Veröffentlichungsnummer: WO 2001/040441

(56) Entgegenhaltungen:
- DE-A- 19 739 364
- US-A- 2 932 128
- FETT W F ET AL: "CHARACTERIZATION OF EXOPOLYSACCHARIDES PRODUCED BY PLANT-ASSOCIATED FLUORESCENT PSEUDOMONADS" APPLIED AND ENVIRONMENTAL MICROBIOLOGY, Bd. 55, Nr. 3, 1989, Seiten 579-583, XP001002048 ISSN: 0099-2240
- LEEMAN M ET AL: "Induction of systemic resistance against fusarium wilt of radish by lipopolysaccharides of Pseudomonas fluorescens." PHYTOPATHOLOGY, Bd. 85, Nr. 9, 1995, Seiten 1021-1027, XP001002114 ISSN: 0031-949X
- "PRORADIX-SEED TREATMENT FOR POTATOES" SOURCON-PADENA AG, [Online] September 2000 (2000-09), XP002168107 Gefunden im Internet: <URL:http://www.sourcon-padena.de/home_e/a grar/kartoffel.htm> [gefunden am 2001-05-22]

## Beschreibung

Die vorliegende Erfindung betrifft die Behandlung von Saatgut und Pflanzen mit Bakterien der Gattung Pseudomonas sowie eine hierzu besonders geeignete neue Spezies der Gattung Pseudomonas.

Aus der DE 197 39 364 A1 ist bereits ein Verfahren zur Stärkung und zum Schutz von Pflanzen bekannt, bei dem Mikroorganismen der Gattung Pseudomonas eingesetzt werden.

Bei dem bekannten Verfahren wird mindestens ein Resistenzinduktor zusammen mit einem nützlichen Mikroorganismus in den Pflanzennährboden oder in den Samen der Pflanze eingebracht, wobei Resistenzinduktor und Mikroorganismus eine sich ergänzende positive Wirkung auf die Pflanze aufweisen.

Als Beispiel für einen nützlichen Mikroorganismus ist der Stamm Pseudomonae sp. Psl der Art Pseudomonas fluorescens genannt, der bei der DSMZ hinterlegt ist.

In dieser Druckschrift wird ferner ein Verfahren beschrieben, wie ein derart nützlicher Mikroorganismus aus Erde und Wurzeln isoliert werden kann. Bei diesem Verfahren wird eine Verdünnungsreihe aus Erde und Wurzeln ausplattiert, inkubiert und auf fluoreszierende Kolonien getestet, mit denen dann ein Screening gegen Schadpilze erfolgt. Ein nach dem bekannten Verfahren gefundener Stamm wird dann daraufhin untersucht, inwieweit er mit Resistenzinduktoren kombiniert werden kann. Auf diese Weise wurde der Stamm Pseudomonae sp. PS1 gefunden.

Versuche des Anmelders der vorliegenden Anmeldung haben jedoch ergeben, daß die in der eingangs erwähnten Druckschrift beschriebene Behandlung noch nicht zufriedenstellend ist, was unter anderem darauf zurückzuführen ist, daß teure Resistenzinduktoren zusammen mit dem beschriebenen Mikroorganismus eingesetzt werden müssen, wobei sich ferner ergeben hat, daß die Schutzwirkung häufig nicht zufriedenstellend ist.

Aus der US 2,932,128 ist ein Verfahren zur Behandlung von Saatgut bekannt, bei dem das Saatgut mit einer Bakterienlösung befeuchtet und dann einem Unterdruck ausgesetzt wird, um das Saatgut zu imprägnieren.

Vor diesem Hintergrund liegt der vorliegenden Erfindung die Aufgabe zugrunde, neue Lösungen und Verfahren sowie einen neuen Mikroorganismus zur Behandlung von Saatgut und/oder Pflanzen bereitzustellen.

Erfindungsgemäß wird diese Aufgabe einerseits im wesentlichen gelöst durch einen Pseudomonas-Stamm mit der Bezeichnung Proradix, der am 03.11.1999 unter der Aufnahmenummer Pseudomonas sp. PRORADIX - DSM 13134 bei der DSMZ, 38124 Braunschweig nach dem Budapester Vertrag hinterlegt wurde.

Der Erfinder der vorliegenden Anmeldung hat den Pseudomonas-Stamm Proradix isoliert, der nach Untersuchung der DSMZ wahrscheinlich eine neue Spezies innerhalb der RNA-Gruppe I der Pseudomonadaceae darstellt. Eine endgültige Zuordnung von Proradix konnte jedoch nicht erfolgen, da nur eine relativ geringe Sequenzähnlichkeit zu gültig beschriebenen Arten der Gattung Pseudomonas besteht.

Feldversuche mit der Spezies Proradix haben gezeigt, daß es sich dabei um ein nützliches Bakterium handelt, das die Wurzeln von Nutzpflanzen besiedeln und von dort aus seine Wirkung auf die Pflanze entfalten kann. Dabei hat sich gezeigt, daß eine Anwendung pro Vegetationsperiode ausreicht und daß auf zusätzliche Resistenzinduktoren verzichtet werden kann.

Proradix kann in einer Formulierung als Flüssigbeize oder als Pulver eingesetzt werden und ist geeignet zur Behandlung von Gemüsesaaten, insbesondere von Feldsalat und Möhren, von Rasensaaten und Gehölzsaaten. Ferner kann es bei Gründüngungssaaten zur biologischen Bodenverbesserung und Entseuchung sowie zum Beizen und zur Tauchbehandlung von Kartoffeln eingesetzt werden. Bei all diesen Anwendungen hat Proradix eine Verbesserung der Bonität verglichen mit unbehandelten Kontrollgruppen sowie teilweise auch im Vergleich zu chemischen Fungiziden gezeigt.

Die Erfindung wird ferner im wesentlichen gelöst durch ein Verfahren zur Herstellung einer Lösung zur Behandlung von Pflanzen und/oder Saatgut, mit den Schritten:
a) Bereitstellen eines Isolates von Bakterien der Gattung Pseudomonas, die den Pseudomonas-Stamm Proradix aufweisen,
b) Bereitstellen eines Nährmediums, in dem Phosphor- und Stickstoffverbindungen sowie Bernsteinsäure enthalten sind,
c) Animpfen des Nährmediums mit dem Isolat, und
d) Inkubation des Nährmediums bei ca. 26-32 °C unter leichtem Schütteln für mindestens 50 Stunden.

Der Erfinder hat erkannt, daß eine derart hergestellte Lösung zu einer Konditionierung der nützlichen Bakterien führt, die zu einer verbesserten Schutzwirkung bei den damit behandelten Pflanzen/dem behandelten Saatgut beiträgt. Diese Konditionierung besteht nach vorläufiger Einschätzung des Erfinders in der Induzierung der Bildung von Exopolisacchariden durch die Abbauprodukte der Bernsteinsäure. Die Exopolisaccharide sind eine Schutzsubstanz, die einen Schleim bilden, durch den die Bakterien besonders gut auf dem Saatgut/den Pflanzen lagerfähig werden.

Besonders bevorzugt ist es dabei, wenn das Anzuchtmedium der Bakterien K₂HPO₄, KH₂PO₄, (NH₄) ₂SO₄, MgSO₄ und Bernsteinsäure enthält, wobei vorzugsweise 6,0 g K₂HPO₄, 3,0 g KH₂PO₄, 1,0 g (NH₄) ₂SO₄, 0,2 g MgSO₄ und 4,0 g Bernsteinsäure in 1000 ml deionisiertem H₂O enthalten sind.

Der Erfinder hat erkannt, daß in diesem Medium besonders schnell Exopolisaccharide gebildet werden, wobei die Zelldichte für die Saatgutbehandlung bei einer nach diesem Verfahren hergestellten Lösung nicht so entscheidend ist wie die bereits erwähnte Konditionierung.

Andererseits ist es bevorzugt, wenn das Nährmedium Glucose enthält, wobei vorzugsweise 6,0 g K₂HPO₄, 3,0 g KH₂PO₄, 1,5 g (NH₄) ₂SO₄, 0,2 g MgSO₄, 2,0 g Glucose und 4 g Bernsteinsäure in 1000 ml deionisiertem Wasser enthalten sind.

Bei dieser Zusammensetzung des Nährmediums kann eine höhere Zelldichte erreicht werden, weil eine C-Quelle in höherer Konzentration vorhanden ist. Der Erfinder hat jedoch erkannt, daß nur durch Glucose im Nährmedium schnell ein saurer pH-Wert entstehen wurde, der eine weitere Kultivierung nicht ermöglicht.

Deswegen wird eine Mischung aus Glucose und Bernsteinsäure als C-Quelle gewählt, so daß zuerst die Glucose umgesetzt wird, wodurch eine hohe Zelldichte aber ein niedriger pH-Wert entsteht. Nach dem Verbrauch der Glucose wird die Bernsteinsäure umgesetzt, wodurch der pH-Wert steigt, so daß ein Überleben aber ein langsameres Wachstum der Bakterien erfolgt. Dies führt zu der bereits erwähnten Exopolisaccharidbildung mit der einhergehenden verbesserten Wirkung von in einer derartigen Lösung angezogenen Mikroorganismen.

Dabei wird ein Isolat der Spezies Proradix eingesetzt.

Die Erfindung betrifft ferner eine nach dem insoweit beschriebenen Verfahren hergestellte Lösung, die Bakterien der Gattung Pseudomonas in einem Nährmedium aufweist, in dem Phosphor- und Stickstoffverbindungen sowie Bernsteinsäure enthalten sind. Nach einer bevorzugten Ausgestaltung enthält das Nährmedium K₂HPO₄, KH₂PO₄, (NH₄)₂SO₄, MgSO₄ und Bernsteinsäure. Nach einer weiteren bevorzugten Ausgestaltung enthält das Nährmedium Glucose. Nach einer weiteren bevorzugten Ausgestaltung enthält das Nährmedium 6,0 g K₂HPO₄, 3,0 g KH₂PO₄, 1,0 g (NH₄)₂SO₄, 0,2 g MgSO₄ und 4,0 g Bernsteinsäure in 1000 ml deionisiertem Wasser. Nach einer weiteren bevorzugten Ausgestaltung enthält das Nährmedium 6,0 g K₂HPO₄, 3,0 g KH₂PO₄, 1,5 g (NH₄)₂SO₄, 0,2 g MgSO₄, 2,0 g Glucose und 4,0 g Bernsteinsäure in 1000 ml deionisiertem Wasser. Dabei handelt es sich bei dem Bakterium der Gattung Pseudomonas um den Pseudomonas-Stamm mit der Bezeichnung Proradix, der am 03.11.1999 unter der Aufnahmenummer PRORADIX - DSM 13134 bei der DSMZ, 38124 Braunschweig nach dem Budapester Vertrag hinterlegt wurde.

Andererseits ist es auch möglich, die Lösung vakuumzutrocknen, was insbesondere dann von Vorteil ist, wenn das Nährmedium Glucose enthält, so daß in dem entstehenden Pulver eine hohe Bakteriendichte enthalten ist. Dieses Pulver läßt sich dann beim Feldeinsatz in Wasser lösen, wobei das so behandelte Wasser dann zum Besprühen von Saatgut, Kartoffeln etc. eingesetzt werden kann.

Vor diesem Hintergrund betrifft die vorliegende Erfindung ebenfalls ein derart hergestelltes Pulver, das Bakterien der Gattung Pseudomonas und ggf. Formulierungshilfsstoffe wie Magermilchpulver und Gummi Arabicum aufweist, wobei es sich bei dem Bakterium der Gattung Pseudomonas um den Pseudomonas-Stamm mit der Bezeichnung Proradix handelt, der am 03.11.1999 unter der Aufnahmenummer PRORADIX - DSM 13134 bei der DSMZ, 38124 Braunschweig nach dem Budapester Vertrag hinterlegt wurde.

Ferner betrifft die Erfindung ein Verfahren zur Behandlung von Saatgut, bei dem mit der neuen Lösung befeuchtetes Saatgut vorübergehend einem Unterdruck ausgesetzt wird.

Der Erfinder der vorliegenden Anmeldung hat nämlich erkannt, daß insbesondere nach dem neuen Verfahren konditionierte Bakterien der Spezies Proradix für eine Vakuumbehandlung von Saatgut geeignet sind und zu hervorragenden Schutzergebnissen führen.

Andererseits betrifft die Erfindung auch ein Verfahren, bei dem das Saatgut/die Pflanzen mit einer Lösung des neuen Pulvers in Wasser besprüht werden. Auch dieses einfache Verfahren führt zu einem effizienten Schutz der behandelten Pflanzen.

Schließlich betrifft die Erfindung noch die Verwendung des Pseudomonas-Stammes Proradix zur Behandlung von Pflanzen sowie Saatgut, das nach dem neuen Verfahren oder gemäß der neuen Verwendung behandelt wurde und den Pseudomonas-Stamm Proradix enthält. ,

Weitere Vorteile ergeben sich aus der nachstehenden Beschreibung von Beispielen für die Anzucht und Verwendung des neuen Verfahrens sowie der Spezies Proradix.

Es versteht sich, daß die vorstehend genannten und die nach-stehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

### Beispiel 1: Isolation der Spezies Proradix

Erdproben und Wurzeln wurden aufgeschwemmt und in Verdünnungsreihen auf einem selektiven Medium ausplattiert, so daß Einzelkolonien erhalten wurden. Fluoreszierende Kolonien wurden für ein in vitro Screening eingesetzt, bei dem mehr als 500 Isolate darauf getestet wurden, ob phytopathogene Pilze, insbesondere der Bodenpilz Rhizoctonia, am Wachstum gehemmt werden. Interessante Kandidaten wurden ausgewählt, an Pflanzen sowie anschließend im Feldversuch getestet.

Bei diesen Tests hat der Erfinder der vorliegenden Anmeldung die Spezies Proradix (DSM 13134) der Gattung Pseudomonas isoliert, die gemäß Identifizierung der DSMZ eine neue Spezies innerhalb der RNA-Gruppe I der Pseudomonadaceae ist. Eine endgültige Zuordnung konnte jedoch nicht erfolgen, da nur eine relativ geringe Sequenzähnlichkeit zu gültig beschriebenen Arten besteht, es handelt sich jedoch zweifelsfrei um einen fluoreszierenden Vertreter der RNA-Gruppe 1 der Pseudomonaden.

### Beispiel 2: Anzucht des Isolates

a) Zur Saatgutbehandlung wird ein Isolat von Pseudomonaden, vorzugsweise der Spezies Proradix, in folgendes Nährmedium inokuliert:

| | |
|---|---|
| K₂HPO₄ | 6,0 g |
| KH₂PO₄ | 3,0 g |
| (NH₄)₂SO₄ | 1,0 g |
| MgSO₄ | 0,2 g |
| Bernsteinsäure | 4,0 g |

auf 1.000 ml deionisiertes H₂O aufgefüllt
Das angeimpfte Nährmedium wird bei 28 °C/100 rpm für 72 Stunden inkubiert, es entsteht eine "Flüssigbeize".
b) Zur Erzeugung einer pulverförmigen Formulierung wird ein Isolat von Pseudomonaden, vorzugsweise der Spezies Proradix, in folgendem Nährmedium kultiviert:

| | |
|---|---|
| K₂HPO₄ | 6,0 g |
| KH₂PO₄ | 3,0 g |
| (NH₄)₂SO₄ | 1,5 g |
| MgSO₄ | 0,2 g |
| Glucose | 2,0 g |
| Bernsteinsäure | 4,0 g |

auf 1.000 ml deionisiertes H₂O aufgefüllt
Das so angeimpfte Nährmedium wird bei 28 - 30 °C/100 rpm je nach Stärke des Inokulums für 65 - 70 Stunden inkubiert.
Anschließend wird die Lösung in einem Vakuumtrockenschrank pulverisiert. Formulierungshilfsstoffe: Magermilchpulver und Gummi-Arabicum.
c) Durch beide Nährmedien wird die Bildung von Exopolisacchariden induziert, wobei bei dem unter a) beschriebenen Nährmedium diese Bildung sehr schnell erfolgt, die Zelldichte ist hier nicht entscheidend, da für die Saatgutbehandlung nicht große Mengen an Bakterien benötigt werden.
Bei dem unter b) beschriebenen Medium ist jedoch eine hohe Zelldichte erforderlich, wobei trotzdem eine Exopolisaccharidbildung einsetzen soll. Dies wird durch die Mischung aus Glucose und Bernsteinsäure erreicht. Zuerst wird die Glucose umgesetzt, was zu einer Erniedrigung des pH-Wertes aber zu einer hohen Zelldichte führt. Wenn die Glucose verbraucht ist, wird die Bernsteinsäure als C-Quelle verwendet, wobei deren Abbauprodukte zu einem Ansteigen des pH-Wertes und zu einer Exopolisaccharidbildung führen.

### Beispiel 3: Behandlung von Saatgut mit Proradix

Eine Möglichkeit der Behandlung besteht darin, das gemäß Beispiel 2b) hergestellte Pulver in Wasser zu lösen und das Saatgut bzw. die Jungpflanzen vor der Ausbringung damit zu besprühen.

Ferner ist es auch möglich, die mit dem in Beispiel 2a) beschriebenen Nährmedium hergestellte Lösung direkt auf Saatgut/Pflanzen vor deren Ausbringung aufzusprühen.

Wenn das behandelte Saatgut noch zwischengelagert werden soll, wird es zunächst mit der in Beispiel 2a) beschriebenen Lösung befeuchtet, dann in eine verschließbare Kammer eingeführt, in der dann vorübergehend ein Unterdruck erzeugt wird.

Auf diese Weise dringen die Bakterien in das Saatgut ein und können auch nach längerer Lagerung ihre Schutzwirkung noch effizient entfalten.

### Beispiel 4: Behandlung von Salat-Jungpflanzen der Sorte Garunda

Über Jungpflanzen der Sorte Garunda, die auf ein Hektar Ackerland ausgebracht werden sollen, wird eine Lösung von 50 ml Proradix (10⁷ cfu/ml) pro Pflanze (gemäß Beispiel 2b) gegossen, bevor die Jungpflanzen auf das Feld kommen.

Nach der Ernte waren lediglich 50 % der Pflanzen nicht marktfähig, während bei einer unbehandelten Kontrolle 70 % der geernteten Pflanzen nicht marktfähig waren.

Bei einer zum Vergleich durchgeführten Behandlung mit den chemischen Fungiziden Switsch (NOVARTIS) (0,8 kg/ha) und Risolex (Spiess Urania) (3 1/ha) waren 58 bzw. 48 % nicht marktfähig.

Dieses Ergebnis zeigt, daß der Salat durch Behandlung mit Proradix deutlich weniger anfällig gegen Schwarzfäule wird, wobei die Ergebnisse mindestens so gut sind wie bei der Behandlung mit chemischen Fungiziden.

### Beispiel 5: Behandlung von Kartoffeln

Proradix wird pulverförmig formuliert, wie in Beispiel 2b) beschrieben, und das Pulver wie folgt in Wasser gelöst:
60 g Pulver in 80 1 Wasser
   für 1 ha Pflanzkartoffeln
60g Pulver =̂ 4x10¹² cfu

Mit dieser Lösung werden die Kartoffelknollen maschinell besprüht und direkt anschließend gelegt. Alternativ werden die Kartoffelknollen direkt mit dem Pulver bestäubt.

Vergleichsversuche zwischen unbehandelten (Kontrolle), mit Proradix behandelten und mit FZB24 (Bayer Leverkusen, Basis: Bacillus subtilis) behandelten Kartoffeln ergaben etwa gleiche Hektarerträge an Marktware. Die Bonitur zeigte jedoch bei einer Behandlung mit Proradix einen zuverlässigen Schutz vor der Braunfleckenkrankheit (Rhizoctonia-Befall), ca. 45 % der geernteten Kartoffeln waren ohne und weitere ca. 45 % mit leichtem Befall. In der Kontrollgruppe betrugen die entsprechenden Zahlen 10 % und 50 %, bei FZB24 25 % und 50 %.

Auch hier zeigt sich die Überlegenheit von Proradix gegenüber herkömmlichen Pflanzenschutzmitteln.

### Beispiel 6: Behandlung von Waschmöhren

Möhrensaatgut wird wie in Beispiel 3 beschrieben mit Proradix-Beize aus Beispiel 2a) befeuchtet und unter Vakuum behandelt (Saatgutinfiltration), zwischengelagert und ausgesät.

Die geernteten Möhren wurden für vier Monate bei 4 °C eingelagert, dann - wie beim Verkauf üblich - zwei Wochen bei Raumtemperatur gelagert und schließlich bewertet.

Die Bonitur ergab bei der Sorte Nerac 50 % ohne Mängel (unbehandelte Kontrolle: 18 %) und 27 % unbrauchbare Möhren (Kontrolle: 58 %). Bei der Sorte Mocum betrugen die Zahlen 36 % (Kontrolle: 14 %) bzw. 46 % (Kontrolle: 54 %).

Die Behandlung von Waschmöhren mit Proradix führt also zu einer deutlichen Steigerung der Erntequalität.

Zusammenfassend läßt sich also sagen, daß zur Behandlung von Pflanzen und/oder Saatgut ein Isolat von nützlichen Bakterien, vorzugsweise der Gattung Pseudomonas, vorzugsweise der Spezies Proradix, bereitgestellt und in einem Nährmedium inkubiert wird, das Phosphor- und Stickstoffverbindungen sowie Bernsteinsäure enthält. Die Lösung kann unmittelbar zum Besprühen von Pflanzen und/oder Saatgut verwendet werden, wobei wahlweise eine Vakuumbehandlung nachgeschaltet werden kann. Die Lösung kann ferner vakuumgetrocknet werden, wobei das Pulver vor der Anwendung in Wasser gelöst wird.

## Patentansprüche

1. Pseudomonas-Stamm mit der Bezeichnung Proradix, der am 03.11.1999 unter der Aufnahmenummer PRORADIX - DSM 13134 bei der DSMZ, 38124 Braunschweig nach dem Budapester Vertrag hinterlegt wurde.

2. Verfahren zur Herstellung einer Lösung zur Behandlung von Pflanzen und/oder Saatgut, mit den Schritten:
a) Bereitstellen eines Isolates von Bakterien der Gattung Pseudomonas,
b) Bereitstellen eines Nährmediums, in dem Phosphor- und Stickstoffverbindungen sowie Bernsteinsäure enthalten sind,
c) Animpfen des Nährmediums mit dem Isolat, und
d) Inkubation des Nährmediums bei ca. 26-32 °C unter Schütteln für mindestens 50 Stunden,
**dadurch gekennzeichnet, dass** die Bakterien den Pseudomonas-Stamm nach Anspruch 1 aufweisen.

3. Verfahren nach Anspruch 2, **dadurch gekennzeichnet, daß** das Nährmedium K₂HPO₄, KH₂PO₄, (NH₄)₂SO₄, MgSO₄ und Bernsteinsäure enthält.

4. Verfahren nach Anspruch 2 oder 3, **dadurch gekennzeichnet, daß** das Nährmedium Glucose enthält.

5. Verfahren nach Anspruch 3, **dadurch gekennzeichnet, daß** das Nährmedium 6,0 g K₂HPO₄, 3,0 g KH₂PO₄, 1,0 g (NH₄)₂SO₄, 0,2 g MgSO₄ und 4,0 g Bernsteinsäure in 1000 ml deionisiertem Wasser enthält.

6. Verfahren nach Anspruch 4, **dadurch gekennzeichnet, daß** das Nährmedium 6,0 g K₂HPO₄, 3,0 g KH₂PO₄, 1,5 g (NH₄)₂SO₄, 0,2 g MgSO₄, 2,0 g Glucose und 4,0 g Bernsteinsäure in 1000 ml deionisiertem Wasser enthält.

7. Verfahren nach Anspruch 6, **dadurch gekennzeichnet, daß** die Lösung nach der Inkubation vakuumgetrocknet wird, um ein Pulver herzustellen.

8. Lösung zur Behandlung von Pflanzen und/oder Saatgut, die Bakterien der Gattung Pseudomonas in einem Nährmedium aufweist, in dem Phosphor- und Stickstoffverbindungen sowie Bernsteinsäure enthalten sind, **dadurch gekennzeichnet, daß** die Bakterien den Pseudomonas-Stamm nach Anspruch 1 aufweisen.

9. Lösung nach Anspruch 8, **dadurch gekennzeichnet, daß** das Nährmedium K₂HPO₄, KH₂PO₄, (NH₄)₂SO₄, MgSO₄ und Bernsteinsäure enthält.

10. Lösung nach Anspruch 8 oder 9, **dadurch gekennzeichnet, daß** das Nährmedium Glucose enthält.

11. Lösung nach Anspruch 10, **dadurch gekennzeichnet, daß** das Nährmedium 6,0 g K₂HPO₄, 3,0 g KH₂PO₄, 1,0 g (NH₄)₂SO₄, 0,2 g MgSO₄ und 4,0 g Bernsteinsäure in 1000 ml deionisiertem Wasser enthält.

12. Lösung nach Anspruch 11, **dadurch gekennzeichnet, daß** das Nährmedium 6,0 g K₂HPO₄, 3,0 g KH₂PO₄, 1,5 g (NH₄)₂SO₄, 0,2 g MgSO₄, 2,0 g Glucose und 4,0 g Bernsteinsäure in 1000 ml deionisiertem Wasser enthält.

13. Pulver zur Behandlung von Pflanzen und/oder Saatgut, das den Pseudomonas-Stamm nach Anspruch 1 aufweist.

14. Pulver nach Anspruch 13, **dadurch gekennzeichnet, daß** das Pulver Formulierungshilfsstoffe aufweist.

15. Pulver nach Anspruch 14, **dadurch gekennzeichnet, daß** die Formulierungshilfsstoffe Magermilchpulver und Gummi-Arabicum aufweisen.

16. Verfahren zur Behandlung von Saatgut/Pflanzen, mit den Schritten:
a) Befeuchten des Saatgutes/der Pflanzen mit der Lösung nach einem der Ansprüche 8 bis 12,
b) Anlegen eines Unterdruckes an das so befeuchtete Saatgut/die so befeuchteten Pflanzen, und
c) Abbau des Unterdruckes.

17. Verfahren zur Behandlung von Saatgut/Pflanzen, mit den Schritten:
a) Auflösen des Pulvers nach einem der Ansprüche 13 bis 15 in Wasser, und
b) Besprühen bzw. Tauchbehandlung des Saatgutes/der Pflanzkartoffeln oder Übergießen von Jungpflanzen mit der Lösung aus Schritt a).

18. Verwendung des Pseudomonas-Stammes Proradix aus Anspruch 1 zur Behandlung von Pflanzen und/oder Saatgut.

19. Nach dem Verfahren aus Anspruch 16 oder Anspruch 17 oder nach der Verwendung aus Anspruch 18 behandeltes Saatgut, das den Pseudomonas-Stamm nach Anspruch 1 enthält.

## Claims

1. Pseudomonas strain designated as Proradix, which was deposited on 03.11.1999 under the entry no. PRORADIX - DSM 13134 at the DSMZ, 38124 Braunschweig, according to the Budapest Treaty.

2. Method for the manufacture of a solution for the treatment of plants and/or seeds, comprising the steps:
a) providing an isolate of bacteria of the genus Pseudomonas,
b) providing a nutrition medium containing phosphor and nitrogen compounds as well as succinic acid,
c) inoculating the nutrition medium with the isolate, and
d) incubation of the nutrition medium at approx. 26-32 °C under agitation for at least 50 hours,
**characterized in that** the bacteria comprise the Pseudomonas strain of claim 1.

3. Method of claim 2, **characterized in that** the nutrition medium contains K₂HPO₄, KH₂PO₄, (NH₄)₂SO₄, MgSO₄ and succinic acid.

4. Method of claim 2 or 3, **characterized in that** the nutrition medium contains glucose.

5. Method of claim 3, **characterized in that** the nutrition medium contains 6.0 g K₂HPO₄, 3.0 g KH₂PO₄, 1.0 g (NH₄)₂SO₄, 0.2 g MgSO₄ and 4.0 g succinic acid in 1000 ml de-ionised water.

6. Method of claim 4, **characterized in that** the nutrition medium contains 6.0 g K₂HPO₄, 3.0 g KH₂PO₄, 1.5 g (NH₄)₂SO₄, 0.2 g MgSO₄, 2.0 g glucose and 4.0 g succinic acid in 1000 ml de-ionised water.

7. Method of claim 6, **characterized in that** the solution is dried after the incubation under vacuum for manufacturing a powder.

8. Solution for the treatment of plants and/or seeds, which comprises bacteria of the genus Pseudomonas in a nutrition medium, which contains phosphor and nitrogen compounds as well as succinic acid, **characterized in that** the bacteria comprise the Pseudomonas strain of claim 1.

9. Solution of claim 8, **characterized in that** the nutrition medium contains K₂HPO₄, KH₂PO₄, (NH₄)₂SO₄, MgSO₄ and succinic acid.

10. Solution of claim 8 or 9, **characterized in that** the nutrition medium contains glucose.

11. Solution of claim 10, **characterized in that** the nutrition medium contains 6.0 g K₂HPO₄, 3.0 g KH₂PO₄, 1.0 g (NH₄)₂SO₄, 0.2 g MgSO₄ and 4.0 g succinic acid in 1000 ml de-ionised water.

12. Solution of claim 11, **characterized in that** the nutrition medium contains 6.0 g K₂HPO₄, 3.0 g KH₂PO₄, 1.5 g (NH₄)₂SO₄, 0.2 g MgSO₄, 2.0 g glucose and 4.0 g succinic acid in 1000 ml de-ionised water.

13. Powder for the treatment of plants and/or seeds comprising the Pseudomonas strain of claim 1.

14. Powder of claim 13, **characterized in that** the powder comprises formulation adjuvants.

15. Powder of claim 14, **characterized in that** the formulation adjuvants comprise low-fat milk powder and gum arabic.

16. Method for the treatment of seeds/plants, comprising the steps:
a) moisturizing the seeds/plants with the solution of any of claims 8 to 12,
b) applying a sub-pressure to the moisturized seeds/plants, and
c) declining the sub-pressure.

17. Method for the treatment of seeds/plants, comprising the steps:
a) desolving the powder of any of claims 13 to 15 in water, and
b) spraying or immersing the seeds/seed potatoes or dousing of young plants with or into the solution of step a).

18. Use of the Pseudomonas strain Proradix of claim 1 for the treatment of plants and/or seeds.

19. Seeds treated by the method of claim 16 or claim 17 or by the use of claim 18, containing the Pseudomonas strain of claim 1.

## Revendications

1. Souche de Pseudomonas portant le nom de Proradix, qui a été déposée le 03.11.1999 sous la référence d'enregistrement PRORADIX - DSM 13134 auprès de la DSMZ, 38124 Braunschweig selon le traité de Budapest.

2. Procédé de production d'une solution pour le traitement de plantes et/ou de semences, comportant les étapes de :
a) mise à disposition d'un isolat de bactéries du genre Pseudomonas,
b) mise à disposition d'un milieu nutritif dans lequel sont contenus des composés de phosphore et d'azote et de l'acide succinique,
c) inoculation du milieu nutritif avec l'isolat, et
d) incubation du milieu nutritif à environ 26 à 32° C en agitant pendant au moins 50 secondes,
**caractérisée en ce que** les bactéries présentent la souche de Pseudomonas selon la revendication 1.

3. Procédé selon la revendication 2, **caractérisé en ce que** le milieu nutritif contient du K₂HPO₄, du KH₂PO₄, du (NH₄)₂SO₄, du MgSO₄ et de l'acide succinique.

4. Procédé selon la revendication 2 ou 3, **caractérisé en ce que** le milieu nutritif contient du glucose.

5. Procédé selon la revendication 3, **caractérisé en ce que** le milieu nutritif contient 6,0 g de K₂HPO₄, 3,0 g de KH₂PO₄, 1,0 g de (NH₄)₂SO₄, 0,2 g de MgSO₄ et 4,0 g d'acide succinique dans 1000 ml d'eau désionisée.

6. Procédé selon la revendication 4, **caractérisé en ce que** le milieu nutritif contient 6,0 g de K₂HPO₄, 3,0 g de KH₂PO₄, 1,5 g de (NH₄)₂SO₄, 0,2 g de MgSO₄, 2,0 g de glucose et 4,0 g d'acide succinique dans 1000 ml d'eau désionisée.

7. Procédé selon la revendication 6, **caractérisé en ce que** la solution est séchée sous vide après l'incubation, pour produire une poudre.

8. Solution pour le traitement de plantes et/ou de semences, qui présente des bactéries du genre Pseudomonas dans un milieu nutritif, dans lequel sont contenus des composés de phosphore et d'azote et de l'acide succinique, **caractérisée en ce que** les bactéries présentent la souche de Pseudomonas selon la revendication 1.

9. Solution selon la revendication 8, **caractérisée en ce que** le milieu nutritif contient du K₂HPO₄, du KH₂PO₄, du (NH₄)₂SO₄, du MgSO₄ et de l'acide succinique.

10. Solution selon la revendication 8 ou 9, **caractérisée en ce que** le milieu nutritif contient du glucose.

11. Solution selon la revendication 10, **caractérisée en ce que** le milieu nutritif contient 6,0 g de K₂HPO₄, 3,0 g de KH₂PO₄, 1,0 g de (NH₄)₂SO₄, 0,2 g de MgSO₄ et 4,0 g d'acide succinique dans 1000 ml d'eau désionisée.

12. Solution selon la revendication 11, **caractérisée en ce que** le milieu nutritif contient 6,0 g de K₂HPO₄, 3,0 g de KH₂PO₄, 1,5 g de (NH₄)₂SO₄, 0,2 g de MgSO₄, 2,0 g de glucose et 4,0 g d'acide succinique dans 1000 ml d'eau désionisée.

13. Poudre pour le traitement de plantes et/ou de semences, qui présente la souche de Pseudomonas selon la revendication 1.

14. Poudre selon la revendication 13, **caractérisée en ce que** la poudre présente un auxiliaire de formulation.

15. Poudre selon la revendication 14, **caractérisée en ce que** les auxiliaires de formulation présentent du lait écrémé en poudre et de la gomme arabique.

16. Procédé de traitement de semences/plantes, comportant les étapes de:
a) humidification de la semence/des plantes avec la solution selon l'une des revendications 8 à 12,
b) application d'une pression négative à la semence ainsi humidifiée/aux plantes ainsi humidifiées, et
c) arrêt de la pression négative.

17. Procédé de traitement de semences/plantes comportant les étapes de :
a) dissolution de la poudre selon l'une des revendications 13 à 15 dans l'eau, et
b) pulvérisation ou traitement par immersion de la semence/ des plants de pomme de terre ou arrosage de jeunes plants avec la solution de l'étape a).

18. Utilisation de la souche de Pseudomonas Proradix selon la revendication 1, pour le traitement de plantes et/ou de semences.

19. Semences traitées avec le procédé selon la revendication 16 ou la revendication 17 ou conformément à l'utilisation selon la revendication 18, qui contiennent la souche de Pseudomonas selon la revendication 1.
